# EUROPEAN PATENT APPLICATION

(11) **EP 4 394 090 A1**
(43) Date of publication of application: **03.07.2024**
(21) Application number: 23220020.4
(22) Date of filing: 22.12.2023
(51) Int. Cl.: C25D 3/48, C25D 5/50, C25D 7/12

(54) **ELECTROPLATING GOLD PLATING SOLUTION AND USE THEREOF**

(30) Priority: 29.12.2022 CN 202211706566
(71) Applicant: Huawei Technologies Co., Ltd., Longgang Shenzhen, Guangdong 518129 (CN); Shenzhen United Blue Ocean Applied Materials Technology Co., Ltd., Shenzhen, Guangdong 518020 (CN)
(72) Inventor: REN, Changyou, Shenzhen, 518129 (CN); WANG, Tong, Shenzhen, 518020 (CN)
(74) Representative: Nederlandsch Octrooibureau

(57) **Abstract**

Embodiments of the present invention provide an electroplating gold plating solution and use thereof. The electroplating gold plating solution comprises a gold cyanide salt, a conductive salt, an oxalate, a lead-containing compound, and additives comprising a phenolic compound and/or gelatin. The general formula of the phenolic compound is as shown in the following formula (1):

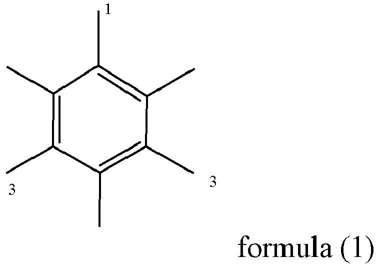

Wherein at least one of R₁, R₂, R₃, R₄, R₅, and R₆ is selected from hydroxyl, and the rest are each independently any one selected from the group consisting of hydrogen atom, halogen group, carboxyl, aldehyde, sulfonic acid group, amino, or alkyl. The technical solution of the present invention can produce the gold bump having a high hardness after heat treatment by adding a phenolic compound and/or gelatin in the electroplating gold plating solution, which is conducive to preventing the gold bump from deformation during the thermocompression bonding process.

## Description

### TECHNICAL FIELD

The present invention relates to the technical field of electroplating gold, and in particular to an electroplating gold plating solution and use thereof, a gold bump and a preparation method thereof, an electronic part, and an electronic device.

### BACKGROUND ART

Gold, as a commonly used metal interconnection material for semiconductors, is widely applied in electronic circuit boards, electronic connectors, semiconductor manufacturing, and the like. For example, gold bumps are commonly used to connect the liquid crystal driver chip and the printed wiring substrate.

Currently, in order to fully exploit the performance of the liquid crystal driver chips to meet the high requirements of the liquid crystal display (LCD) technology development on the display resolution, refresh frequency, brightness, contrast, and the like, the sizes of gold bumps and the gaps between the neighboring gold bumps are designed to be increasingly smaller. However, the reduced size of gold bumps and the spacing between the neighboring gold bumps are prone to cause deformation of the gold bumps during the thermocompression bonding process, resulting in an easy connection between the neighboring gold bumps, thereby triggering the problems of short circuit and failure.

Increasing the hardness of said gold bumps is an important means to avoid the deformation of gold bumps during the thermocompression bonding process. However, in practical use at present, gold bumps are mainly produced with the electroplating gold process, wherein the heat treatment is generally implemented after the electroplating process. Because gold is a low-melting metal, its hardness is easily reduced during the heat treatment, which makes it difficult to obtain gold bumps still having a high hardness after the heat treatment.

### SUMMARY OF THE INVENTION

The present invention provides an electroplating gold plating solution and a use thereof, a gold bump and a preparation method thereof, an electronic part, and an electronic device, by adding gelatin and/or a phenolic compound in the electroplating gold plating solution to prepare gold bumps still having a high hardness after the heat treatment, thereby advantageously avoiding deformation of the gold bumps during the thermocompression bonding process.

In the first aspect, the present invention provides an electroplating gold plating solution comprising: a gold cyanide salt, a conductive salt, an oxalate, a lead-containing compound, and additives including a phenolic compound and/or gelatin, and the general formula of the phenolic compound is as shown in the following formula (1):

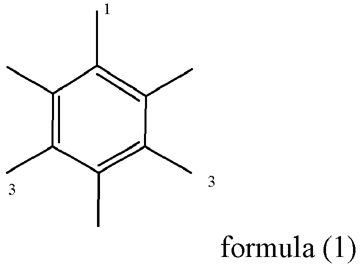

In the formula (1), at least one of R₁, R₂, R₃, R₄, R₅, and R₆ is selected from hydroxyl, and the rest are each independently any one selected from the group consisting of hydrogen atom, halogen group, carboxyl, aldehyde, sulfonic acid group, amino, and alkyl.

In the above-mentioned technical solution, the electroplating gold plating solution is prepared by adding phenolic compounds (e.g., phenol and/or phenol derivatives) and/or gelatin as specific additives, and adding other components to produce synergy, such that the gold bumps prepared with the electroplating gold plating solution can still have a high hardness after the heat treatment (e.g., the hardness of gold bumps after the heat treatment may be within the range of 90-120HV), and the obtained gold bumps have a regular shape. Therefore, the present invention is conducive to avoiding the short circuit and failure caused by the deformation of the gold bumps during the thermocompression bonding process while reducing the size of gold bumps and the spacing between neighboring gold bumps.

Furthermore, compared to the cyanide-free electroplating gold plating solution, the cyanide-based electroplating gold plating solution using a gold cyanide salt as the gold source provided by the examples of the present invention is more suitable for continuous production over a long time (e.g., continuous production for 2-3 years), thus it is more conducive to the industrial application. In addition, the electroplating mode of vertical rack plating used for the cyanide-based electroplating gold plating solution is more advantageous for achieving plating uniformity than the horizontal cup electroplating method used for the cyanide-free electroplating gold plating solution frequently adopted in the production line.

With reference to the first aspect, in some embodiments of the first aspect, the phenolic compound comprises at least one selected from the group consisting of phenol, catechol, resorcinol, hydroquinone, 2-hydroxybenzoic acid, 3-hydroxybenzoic acid, 4-hydroxybenzoic acid, 4-hydroxybenzaldehyde, 4-hydroxybenzenesulfonic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, 3,5-dihydroxybenzoic acid, 3,4,5-trihydroxybenzoic acid, and 2,4, 6-trihydroxybenzoic acid.

In the aforementioned technical solution, by adding phenol or derivative thereof into the electroplating gold plating solution, the gold bumps produced with the electroplating gold plating solution can still have a high hardness after the heat treatment (e.g., the hardness of gold bumps after the heat treatment may be within the range of 90-120HV). Therefore, the present invention is conducive to avoiding the short circuit and failure caused by the deformation of the gold bumps during the thermocompression bonding process while reducing the size of gold bumps and the spacing between neighboring gold bumps.

Regarding the first aspect, in some embodiments of the first aspect, the electroplating gold plating solution satisfies at least one of the following conditions: the concentration of said phenolic compound in the electroplating gold plating solution is greater than or equal to 0.05g/L and less than or equal to 2.0g/L; the concentration of gelatin in the electroplating gold plating solution is greater than or equal to 0.05g/L and less than or equal to 2.0g/L.

In the technical solution, when the concentration of a phenolic compound or gelatin in the electroplating gold plating solution is less than 0.05g/L, due to the low concentration, it is disadvantageous for the phenolic compound or gelatin to play a role in the electroplating gold plating solution, thus it is not conducive to obtaining the gold bumps still having a high hardness after the heat treatment. When the concentration of phenolic compound or gelatin in the electroplating gold plating solution is greater than 2.0g/L, it may result in excessively high hardness of the gold bumps following the heat treatment, and it may also cause a decreased purity of the gold bumps. Therefore, by arranging the concentration range of phenolic compound and gelatin, it is advantageous to ensure that the hardness of gold bumps after the heat treatment is increased without affecting the purity of said gold bumps.

With reference to the first aspect, in some embodiments of the first aspect, the gold cyanide salt comprises at least one of potassium aurous cyanide, sodium aurous cyanide, and ammonium aurous cyanide.

In the aforementioned technical solution, the gold cyanide salt may act as a gold source for the electroplating gold plating solution.

Regarding the first aspect, in some embodiments of the first aspect, the gold cyanide salt is used in an amount such that the concentration of gold ions in the electroplating gold plating solution is greater than or equal to 1g/L and less than or equal to 20 g/L.

In the technical solution, when the concentration of gold ions in the electroplating gold plating solution is less than 1g/L because the concentration of gold ions is too low, it may cause the precipitation efficiency at the cathode to be excessively low, and the other side reactions (e.g., cathodic hydrogen evolution) is serious, which may affect the plating appearance and production efficiency. When the concentration of gold ions in the electroplating gold plating solution is more than 20g/L, although the concentration does not influence the stability of the electroplating gold plating solution and the appearance and physical properties of said plating layer, the precipitation of the metal plating solution is prone to cause waste of gold and finally cause the cost rise. Therefore, by arranging the concentration range of the gold ions in the electroplating gold plating solution, it is advantageous to prevent the increase in cost due to the waste of gold while ensuring the appearance uniformity and the production efficiency of said plating layer of the electroplating gold plating solution.

With reference to the first aspect, in some embodiments of the first aspect, the conductive salt comprises a phosphate and/or an organophosphonic acid; wherein the phosphate comprises at least one of potassium phosphate, sodium phosphate, and ammonium phosphate, the organic phosphonic acid comprises at least one of hydroxyl ethylidene diphosphonic acid, aminotrimethylene phosphonic acid, and ethylenediamine tetramethylene phosphonic acid.

In the technical solution, the conductive salt may be used to improve the conductivity of the electroplating gold plating solution.

Regarding the first aspect, in some embodiments of the first aspect, the concentration of said conductive salt in the electroplating gold plating solution is greater than or equal to 10 g/L and less than or equal to 100 g/L.

In the above technical solution, when the concentration of conductive salt in the electroplating gold plating solution is less than 10g/L, it is apt to cause that the electroplating gold plating solution becomes unstable, the plating uniformity declines and the plating layer becomes coarse. When the concentration of conductive salt in the electroplating gold plating solution is greater than 100g/L, the viscosity of the said electroplating gold plating solution is too large, and the migration of gold ions towards the cathode is likely to be affected, thereby causing roughness of the plating layer. By setting the concentration range of conductive salt in the electroplating gold plating solution, it is advantageous to prevent the problem of a coarse plating layer while improving the conductivity of the electroplating gold plating solution.

With reference to the first aspect, in some embodiments of the first aspect, the oxalate comprises at least one of potassium oxalate, sodium oxalate, and ammonium oxalate.

In the above-mentioned technical solution, the oxalate may serve as an organic conductive salt in the electroplating gold plating solution. When the oxalate is not used in the electroplating gold plating solution, a plating film is easily penetrated between the photoresist and the wafer, thereby causing precipitation outside the pattern. That is, by adding oxalate to the electroplating gold plating solution, it is advantageous to prevent a portion of the electroplating film layer from becoming thick due to the penetration of the plating film, which cannot be completely removed by the etching process of the metallization layer underneath the bump after the gold plating process, thereby causing the phenomenon of defective conduction.

Regarding the first aspect, in some embodiments of the first aspect, the concentration of said oxalate in the electroplating gold plating solution is greater than or equal to 5 g/L and less than or equal to 80 g/L.

In the above technical solution, when the concentration of oxalate in the electroplating gold plating solution is less than 5g/L, because the concentration is excessively low, it is not favorable to the functioning of the oxalate in the electroplating gold plating solution, and it is prone to cause penetration of the plating film. When the concentration of oxalate in the electroplating gold plating solution is greater than 80g/L, it is apt to cause unevenness in the plating film appearance. By arranging the above concentration range of said oxalate in the electroplating gold plating solution, it is advantageous to prevent the plating film from penetration, while ensuring uniformity of the plating film appearance.

With reference to the first aspect, in some embodiments of the first aspect, the lead-containing compound comprises at least one of lead acetate, lead nitrate, lead citrate, and lead sulfate.

In the technical solution, the lead-containing compound may be used as a crystallization conditioning agent in the electroplating gold plating solution to adjust the crystallization of the electroplating gold plating solution.

Regarding the first aspect, in some embodiments of the first aspect, the concentration of said lead-containing compound is calculated in terms of lead, the concentration of said lead-containing compound in the electroplating gold plating solution is greater than or equal to 2ppm and less than or equal to 15ppm.

In the technical solution, by adding a suitable low content of lead-containing compound into the electroplating gold plating solution, the lead-containing compound can play a role in adjusting the crystallinity and hardness of the plating layer and avoiding the reduced purity of the plating layer resulting from the excessive Pb impurities mingled in the formed plating layer.

Concerning the first aspect, in some embodiments of the first aspect, the electroplating gold plating solution further comprises a hydrogen ion concentration index pH additive, which is used for adjusting the pH of the electroplating gold plating solution.

In the technical solution, the pH additive is added to the electroplating gold plating solution to adjust the pH value of the electroplating gold plating solution according to actual production and design requirements.

In one possible embodiment, the pH additive may be an acid or an alkali. The acid may be selected, for example, but not limited to, at least one of sulfuric acid, phosphoric acid, or organic phosphonic acid. Exemplarily, the organic phosphonic acid may be at least one selected from the group consisting of hydroxyl ethylidene diphosphonic acid, aminotrimethylene phosphonic acid, or ethylenediamine tetramethylene phosphonic acid. The alkali may be selected from, for example, but not limited to, at least one of potassium hydroxide, sodium hydroxide, or ammonia water.

Regarding the first aspect, in some embodiments of the first aspect, the pH of said electroplating gold plating solution is greater than or equal to 5 and less than or equal to 7.

In the technical solution, the pH value of the electroplating gold plating solution is set between 5 and 7, on one hand, the lead-containing compound has desirable solubility in the weakly acidic electroplating gold plating solution, it is not easily precipitated out, is beneficial to the effect of avoiding influencing the electroplating gold plating solution, and the electroplating gold plating solution has robust long-term stability. On the other hand, it can favorably avoid the problem that the gold cyanide salt causes volatilization of hydrogen cyanide due to the excessively low pH of the system, such that the gold plating effect is influenced.

In the second aspect, the present invention provides a use of the electroplating gold plating solution according to any item of the first aspect, the electroplating gold plating solution is applied in the gold plating of semiconductors.

In the third aspect, the present invention provides a preparation method for gold bump, the preparation method comprises the following steps: placing a semiconductor in the electroplating gold plating solution according to any item of the first aspect for electroplating; carrying out heat treatment on the electroplated semiconductor to form a gold bump on the surface of said semiconductor.

Regarding the third aspect, in some embodiments of the third aspect, the method satisfies at least one of the following conditions: the electroplating temperature is greater than or equal to 30°C and less than or equal to 70°C; the current density of said electroplating is greater than or equal to 0.1A/dm³ and less than or equal to 1.0A/dm³.

With reference to the third aspect, in some embodiments of the third aspect, the hardness of said gold bump after heat treatment is greater than or equal to 90HV and less than or equal to 120 HV

In the fourth aspect, the present invention provides a gold bump produced with the preparation method according to any one of the third aspects.

In the fifth aspect, the present invention provides an electronic part comprising a gold bump according to the fourth aspect above.

In the sixth aspect, the present invention provides an electronic device comprising the electronic part according to the fifth aspect.

The technical effects of the second aspect to the sixth aspect may be obtained with reference to the related description regarding the technical effects of the first aspect, the details are not repeated herein.

### BRIEF DESCRIPTION OF THE DRAWINGS

FIG. 1 illustrates a 100-fold magnified micrograph of the contour of the gold bump prepared with the electroplating gold plating solution according to Example 1;
FIG. 2 is a graph of the height test results of the gold bump shown in FIG. 1;
FIG. 3 illustrates a 100-fold magnified micrograph of the contour of the gold bump prepared with the electroplating gold plating solution according to Example 10;
FIG. 4 is a graph of the height test results of the gold bump shown in FIG. 3.

### DESCRIPTION OF THE PREFERRED EMBODIMENT

The technical solution in the examples of the present invention will be described below with reference to the accompanying drawings.

The terminology used in the following examples is to describe the particular embodiments only, instead of imposing limitations on the present invention. As used in the description of the present invention and the appended claims, the singular expression forms "a", "an", "the" and "said" are intended to include the plural expression forms such as "one or more", unless otherwise specified in the context. It should also be understood that in the following examples of the present invention, "at least one", and "one or more" refer to one, two, or more. The term "and/or" serves to describe the association relationship of the associated objects, and indicates that there may be three relationships; for example, A and/or B, which may represent three circumstances, namely A alone, both A and B, and B alone, wherein A and B may be singular or plural. The character "/" generally indicates that the former and latter associated objects are in an "or" relationship.

In the description of the present invention, the numerical ranges mentioned herein may include the numerical values at both ends of the range, for example, the hardness within the range of 90-120HV may mean that the hardness is greater than or equal to 90HV and less than or equal to 120 HV In another example, a concentration within the range of 6-17 g/L may refer to a concentration that is greater than or equal to 6 g/L and less than or equal to 17 g/L.

The reference to "an example" or "some examples" as depicted in the description means that a particular feature, structure, or characteristic is described in one or more examples of the present invention with reference to the example(s). Therefore, the phrases "in an example", "in some examples", "in some other examples" and "in other examples" or the like mentioned in various places throughout the description are not necessarily referring to the same examples, but rather mean "one or more examples instead of all the examples", unless otherwise specified and emphasized. The terms "comprising", "including", and "having" and their derivatives are intended to mean "including, but not limited to" unless otherwise specified and emphasized.

Gold has advantages such as desirable chemical stability, low contact resistance, excellent conductivity, good solderability, and thermocompression bonding performance, it is currently a commonly used metal interconnection material for semiconductors and is widely applied in electronic circuit boards, electronic connectors, semiconductor manufacturing, and other fields. For example, gold bumps are commonly used to connect the liquid crystal driver chip and the printed wiring substrate.

However, along with the development of liquid crystal display (LCD) technology, the requirements for display resolution, refresh frequency, brightness, contrast, and the like are increasingly higher, which requires that the liquid crystal driver chip as a control unit can exert higher performance. Therefore, in order to fully exert the performance of the liquid crystal driver chip and ensure access to more input/output units, the sizes of gold bumps and the gaps between the neighboring gold bumps need to be reduced. According to the latest report, the minimum width of the gold bump is 6µm, and the gap between the neighboring gold bumps is 4µm. However, the reduced size of the gold bumps and the gap between the neighboring gold bumps are prone to cause deformation of the gold bumps during the thermocompression bonding process, resulting in a connection between the neighboring gold bumps, thereby triggering the problem of short circuit and failure. It has been discovered in research that the increased hardness of the gold bump can prevent the deformation of gold bumps during the thermocompression bonding process.

In the current practical use, gold bumps are prepared through a gold plating process. To improve the ductility of gold plating, it is often subjected to heat treatment after the electroplating process. The gold plating is generally classified into low hardness (40-60HV), medium hardness (70-90HV), and high hardness (90-120HV) gold plating, depending on the hardness of gold plating after heat treatment. However, gold is a low-melting metal, thus the recrystallization process is likely to occur during the heat treatment process, which causes large crystal grains and reduced hardness, making it difficult to obtain the gold bump having a high hardness, for example, the gold bump having a hardness within the range of 90-120HV after heat treatment.

Based on the above content, the examples of the present invention provide an electroplating gold plating solution and a use thereof, a gold bump and a preparation method thereof, an electronic part, and an electronic device, by adding gelatin and/or a phenolic compound in the electroplating gold plating solution to prepare gold bumps still having a high hardness after the heat treatment (e.g., the hardness of a gold bump after the heat treatment may be 90-120HV), which is beneficial to reduce the size of a gold bump and the gap between adjacent gold bumps, and simultaneously avoiding the short circuit and failure due to the deformation of said gold bumps during the thermalcompression bonding process.

Firstly, the electroplating gold plating solution provided in examples of the present invention is specifically described.

The examples of the application provide an electroplating gold plating solution comprising a gold cyanide salt, a conductive salt, an oxalate, a lead-containing compound, and additives.

Wherein the additives comprise a phenolic compound and/or gelatin, and the general formula of the phenolic compound is as shown in the following formula (1), that is, the phenolic compound may comprise phenol or derivatives thereof.

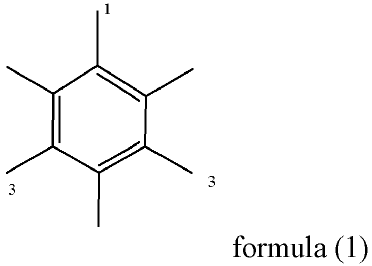

In the formula (1), at least one of R₁, R₂, R₃, R₄, R₅, and R₆ is selected from hydroxyl, and the rest are each independently any one selected from the group consisting of hydrogen atom, halogen group, carboxyl, aldehyde, sulfonic acid group, amino, and alkyl.

The meanings of the atomic groups involved in the above formula (1) are described in detail below. It shall be understood that unless otherwise specified, the following atomic groups have the following meanings. Any undefined atomic group has its meaning generally accepted by those skilled in the art.

In the present invention, the hydroxyl may be a group -OH.

In the present invention, the halogen group may refer to fluorine (F) atom, chlorine (Cl) atom, bromine (Br) atom, or iodine (I) atom. The halogen group may also be substituted by a substituent, and the substituent may include at least one of alkyl, aryl, heteroaryl, and the like, the halogen group is linked to the benzene ring through the substituent.

In the present invention, the carboxyl may refer to a group -COOH. The carboxyl may be substituted by a substituent, the substituent may include at least one of alkyl, aryl, heteroaryl, and the like, the carboxyl is linked to the benzene ring through the substituent.

In the present invention, the aldehyde may refer to a group -CHO. The aldehyde may also be substituted by a substituent, the substituent may include at least one of alkyl, aryl, heteroaryl, and the like, the aldehyde is linked to the benzene ring through the substituent.

In the present invention, the sulfonic acid group may refer to a group -SO₃H. The sulfonic acid group may also be substituted by a substituent, the substituent may include at least one of alkyl, aryl, heteroaryl, and the like, the sulfonic acid group is linked to the benzene ring through the substituent.

In the present invention, the amino may be a group -NH₂. The amino may be substituted or unsubstituted, for example, primary amino, secondary amino, tertiary amino, and the like. Wherein, the substituent of the substituted amino may include at least one of alkyl, aryl, heteroaryl, etc., such as methyl amino, and diphenylamino.

In the present invention, the alkyl may refer to a monovalent saturated aliphatic hydrocarbon group having one or more carbon atoms. The alkyl may be straight or branched. The alkyl may be substituted or unsubstituted, and the number of carbon atoms in the alkyl is not limited. For example, the alkyl may have 1-15 carbon atoms. More preferably, the alkyl has 1-10 carbon atoms. Further preferably, the alkyl has 1-5 carbon atoms. Examples of alkyl may include but are not limited to, methyl, ethyl, propyl, n-propyl, isopropyl, butyl, n-butyl, isobutyl, t-butyl, sec-butyl, 1-methyl-butyl, 1-ethyl-butyl, pentyl, n-pentyl, isopentyl, neopentyl, t-pentyl, hexyl, n-hexyl.

In the above-mentioned technical solution, the electroplating gold plating solution is prepared by adding phenolic compounds (e.g., phenol and/or phenol derivatives) and/or gelatin as specific additives, and adding other components to generate synergy, such that the gold bumps prepared with the electroplating gold plating solution can still have a high hardness after the heat treatment (e.g., the hardness of gold bumps after the heat treatment may be within the range of 90-120HV), and the obtained gold bumps have a regular shape. Therefore, the present invention is conducive to avoiding the short circuit and failure caused by the deformation of the gold bumps during the thermocompression bonding process while reducing the size of gold bumps and the spacing between neighboring gold bumps.

Furthermore, compared to the cyanide-free electroplating gold plating solution, the cyanide-based electroplating gold plating solution using a gold cyanide salt as the gold source provided by the embodiments of the present invention is more suitable for continuous production over a long time (e.g., continuous production for 2-3 years), thus it is more conducive to the industrial application. In addition, the electroplating mode of vertical rack plating used for the cyanide-based electroplating gold plating solution is more advantageous for achieving plating uniformity than the horizontal cup electroplating method used for the cyanide-free electroplating gold plating solution frequently adopted in the production line.

The components included in the electroplating gold plating solution will be specifically described below.

In some examples, the phenolic compound may include phenol and/or a phenol derivative. Exemplarily, the phenolic derivative may include but is not limited to, at least one selected from the group consisting of catechol, resorcinol, hydroquinone, 2-hydroxybenzoic acid, 3-hydroxybenzoic acid, 4-hydroxybenzoic acid, 4-hydroxybenzaldehyde, 4-hydroxybenzenesulfonic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, 3,5-dihydroxybenzoic acid, 3,4,5-trihydroxybenzoic acid, and 2,4, 6-trihydroxybenzoic acid.

In some examples, the electroplating gold plating solution satisfies at least one of the following conditions:
the concentration of said phenolic compound in the electroplating gold plating solution is greater than or equal to 0.05g/L and less than or equal to 2.0 g/L;
The concentration of gelatin in the electroplating gold plating solution is greater than or equal to 0.05 g/L and less than or equal to 2.0 g/L.

It should be noted that, when the concentration of phenolic compound or gelatin in the electroplating gold plating solution is less than 0.05g/L, due to the low concentration, it is disadvantageous for the phenolic compound or gelatin to play a role in the electroplating gold plating solution, thus it is not conducive to obtaining the gold bumps still having a high hardness after the heat treatment. When the concentration of phenolic compound or gelatin in the electroplating gold plating solution is greater than 2.0g/L, it may result in excessively high hardness of the gold bumps following the heat treatment, and it may also cause a decreased purity of the gold bumps. Therefore, by arranging the concentration range of phenolic compound and gelatin, it is advantageous to ensure that the hardness of gold bumps after the heat treatment is increased without affecting the purity of said gold bumps.

Exemplarily, the concentration of the phenolic compound may be 0.05g/L, 0.1g/L, 0.15g/L, 0.25g/L, 0.3g/L, 0.35g/L, 0.4g/L, 0.45g/L, 0.5g/L, 0.7g/L, 0.9g/L, 1.2 g/L, 1.5g/L, 1.8g/L, or 2.0 g/L, as well as a random value within the range consisting of any two of the numerical values. More preferably, the concentration of the phenolic compound may be within the range of 0.8-1.7g/L. Further preferably, the concentration of the phenolic compound may be within the range of 0.9-1.5g/L.

Exemplarily, the concentration of gelatin may be 0.05g/L, 0.11g/L, 0.17g/L, 0.23g/L, 0.29g/L, 0.35g/L, 0.41g/L, 0.47g/L, 0.55g/L, 0.75g/L, 0.95g/L, 1.35g/L, 1.65g/L, or 2.0g/L, as well as a random value within the range consisting of any two of the numerical values. More preferably, the concentration of gelatin may be within the range of 0.75-1.8g/L. Further preferably, the concentration of gelatin may be within the range of 0.9-1.65g/L.

It should be understood that the concentration ranges and specific values of the phenolic compound and gelatin are only illustrative and can be adjusted according to actual production and design requirements, and the present invention is not limited thereto.

In some examples, gold cyanide salt, as the gold source, may include, but is not limited to, at least one of potassium aurous cyanide, sodium aurous cyanide, and ammonium aurous cyanide.

Optionally, the gold cyanide salt is used in an amount such that the concentration of gold ions in the electroplating gold plating solution is greater than or equal to 1 g/L and less than or equal to 20 g/L.

It should be noted that when the concentration of gold ions in the electroplating gold plating solution is less than 1 g/L, because the concentration of gold ions is too low, it may cause the precipitation efficiency at the cathode is excessively low, and the other side reactions (e.g., cathodic hydrogen evolution) is serious, which may affect the plating appearance and production efficiency. When the concentration of gold ions in the electroplating gold plating solution is more than 20 g/L, although the concentration does not influence the stability of the electroplating gold plating solution and the appearance and physical properties of said plating layer, the precipitation of the metal plating solution is prone to cause waste of gold and finally cause the cost rise. Therefore, by arranging the concentration range of the gold ions in the electroplating gold plating solution, it is advantageous to prevent the increase in cost due to the waste of gold while ensuring the appearance uniformity and the production efficiency of said plating layer of the electroplating gold plating solution.

Exemplarily, the concentration of gold ions can be 1g/L, 2g/L, 3g/L, 4g/L, 5g/L, 7g/L, 9g/L, 11g/L, 13g/L, 15g/L, 17g/L, 19g/L, or 20g/L, as well as a random value within the range consisting of any two of the numerical values. More preferably, the concentration of gold ions may be within the range of 6-17g/L. Further preferably, the concentration of gold ions may be within the range of 8-16g/L.

It should be understood that the above-mentioned concentration range values and specific values of gold ions are only illustrative and can be adjusted according to actual production and design requirements, and the present invention is not limited thereto.

In some examples, the conductive salt may include a phosphate and/or an organophosphonic acid for increasing the conductivity of the electroplating gold plating solution. Wherein the organic phosphonic acid may include, but is not limited to at least one of hydroxyl ethylidene diphosphonic acid (HEDP), aminotrimethylene phosphonic acid (ATMP), and ethylenediamine tetramethylene phosphonic acid.

Alternatively, the concentration of the conductive salt in the electroplating gold plating solution may be greater than or equal to 10 g/L, and less than or equal to 100 g/L.

It shall be noted that, when the concentration of conductive salt in the electroplating gold plating solution is less than 10g/L, it is apt to cause that the electroplating gold plating solution becomes unstable, the plating uniformity declines, and the plating layer becomes coarse. When the concentration of conductive salt in the electroplating gold plating solution is greater than 100g/L, the viscosity of the said electroplating gold plating solution is too large, and the migration of gold ions towards the cathode is likely to be affected, thereby causing roughness of the plating layer. By setting the concentration range of conductive salt in the electroplating gold plating solution, it is advantageous to prevent the problem of a coarse plating layer while improving the conductivity of the electroplating gold plating solution.

Exemplarily, the concentration of said conductive salt may be 10g/L, 15g/L, 20g/L, 25g/L, 30g/L, 40g/L, 50g/L, 60g/L, 70g/L, 85g/L, or 100g/L, as well as a random value within the range consisting of any two of the numerical values. More preferably, the concentration of said conductive salt may be within the range of 20-80g/L. Further preferably, the concentration of said conductive salt may be within the range of 35-70g/L.

It should be comprehended that the above-mentioned concentration range values and specific values of conductive salt are only illustrative and can be adjusted according to actual production and design requirements, and the present invention is not limited thereto.

In some examples, the oxalate acts as an organic acid conductive salt, which may include but is not limited to, at least one of potassium oxalate, sodium oxalate, and ammonium oxalate.

It should be noted that, when the oxalate is not used in the electroplating gold plating solution, a plating film is easily penetrated between the photoresist and the wafer, thereby causing precipitation outside the pattern. That is, by adding oxalate to the electroplating gold plating solution, it is advantageous to prevent a portion of the electroplating film layer from becoming thick due to the penetration of the plating film, which cannot be completely removed by the etching process of the metallization layer underneath the bump after the gold plating process, thereby causing the phenomenon of defective conduction.

Optionally, the concentration of said oxalate in the electroplating gold plating solution may be greater than or equal to 5 g/L and less than or equal to 80 g/L.

It should be noted that, when the concentration of oxalate in the electroplating gold plating solution is less than 5g/L, because the concentration is excessively low, it is not favorable to the functioning of the oxalate in the electroplating gold plating solution, and it is prone to cause penetration of the plating film. When the concentration of oxalate in the electroplating gold plating solution is greater than 80g/L, it is apt to cause unevenness in the plating film appearance. By arranging the above concentration range of said oxalate in the electroplating gold plating solution, it is advantageous to prevent the plating film from penetration, while ensuring uniformity of the plating film appearance.

Exemplarily, the concentration of said oxalate may be 5g/L, 10g/L, 15g/L, 20g/L, 25g/L, 35g/L, 45g/L, 55g/L, 70g/L, or 80g/L, as well as a random value within the range consisting of any two of the numerical values. More preferably, the concentration of said oxalate may be within the range of 13-75g/L. Further preferably, the concentration of said oxalate may be within the range of 30-65g/L.

It should be comprehended that the above-mentioned concentration range values and specific values of oxalate are only illustrative and can be adjusted according to actual production and design requirements, and the present invention is not limited thereto.

In some examples, the lead-containing compound acts as a crystallization modifier, for example, it may include, but is not limited to, at least one of lead acetate, lead nitrate, lead citrate, and lead sulfate.

Optionally, the concentration of said lead-containing compound is calculated in terms of lead, the concentration of said lead-containing compound in the electroplating gold plating solution is greater than or equal to 2ppm and less than or equal to 15ppm.

It should be noted that, by adding a suitable low content of lead-containing compound into the electroplating gold plating solution, the lead-containing compound can play a role in adjusting the crystallinity and hardness of the plating layer and avoiding the reduced purity of the plating layer resulting from the excessive Pb impurities mingled in the formed plating layer.

Exemplarily, the concentration of said lead-containing compound may be 2ppm, 4ppm, 6ppm, 8ppm, 10ppm, 11ppm, 13ppm, or 15ppm, and a random value within the range consisting of any two of the numerical values. More preferably, the concentration of said lead-containing compound may be within the range of 5-12 ppm. Further preferably, the concentration of said lead-containing compound may be within the range of 7.5-12 ppm.

It should be understood that the above-mentioned concentration range values and specific values of said lead-containing compound are only illustrative and can be adjusted according to actual production and design requirements, and the present invention is not limited thereto.

In some examples of the present invention, the electroplating gold plating solution may further comprise a hydrogen ion concentration (pH) additive for adjusting the pH of the electroplating gold plating solution.

In some examples, the pH additive may be an acid or an alkali. The acid may be selected, for example, but not limited to, at least one of sulfuric acid, phosphoric acid, or organic phosphonic acid. Exemplarily, the organic phosphonic acid may be at least one selected from the group consisting of hydroxyl ethylidene diphosphonic acid, aminotrimethylene phosphonic acid, or ethylenediamine tetramethylene phosphonic acid. The alkali may be selected, for example, but not limited to, at least one of potassium hydroxide, sodium hydroxide, or ammonia water.

Optionally, the pH of said electroplating gold plating solution is greater than or equal to 5 and less than or equal to 7.

It should be noted that the pH of the electroplating gold plating solution is set between 5 and 7, on one hand, the lead-containing compound has desirable solubility in the weakly acidic electroplating gold plating solution, it is not easily precipitated out, is beneficial to the effect of avoiding influencing the electroplating gold plating solution, and the electroplating gold plating solution has robust long-term stability. On the other hand, it can favorably avoid the problem that the gold cyanide salt causes volatilization of hydrogen cyanide due to the excessively low pH of the system, such that the gold plating effect is influenced.

Exemplarily, the pH of the electroplating gold plating solution may be 5, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, or 7, and a random value within the range consisting of any two of the numerical values. More preferably, the pH of the electroplating gold plating solution may be within the range of 5.2-6.9.

It should be comprehended that the pH range values and specific values of the electroplating gold plating solution are only illustrative and can be adjusted according to actual production and design requirements, and the present invention is not limited thereto.

In some embodiments provided herein, the preparation method of the electroplating high-emitting solution may comprise: mixing a gold cyanide salt, a conductive salt, an oxalate, a lead-containing compound, additives, and water to obtain the electroplating gold plating solution. Wherein the additives comprise a phenolic compound and/or gelatin.

It should be understood that the present invention does not impose limitations on the preparation method of the electroplating gold plating solution, as long as the preparation method can be used for producing the above-mentioned electroplating gold plating solution.

The examples of the present invention provide a use of the electroplating gold plating solution in the gold plating of semiconductor, the gold bump prepared by using the electroplating gold plating solution still has a high hardness after the heat treatment.

Exemplarily, the electroplating gold plating solution may be applied in fields such as electronic circuit boards, electronic connectors, or semiconductor manufacturing. For example, the electroplating gold plating solution can be used for preparing a gold bump product with high hardness (e.g., 90-120HV) on a semiconductor surface through a photoresist technology, such as a liquid crystal driver chip, an image sensor, a fingerprint sensor.

The examples of the present invention provide a preparation method for gold bump, wherein the method may comprise the following steps: placing a semiconductor in the electroplating gold plating solution for electroplating; and then carrying out heat treatment on the electroplated semiconductor to form a gold bump on the surface of said semiconductor.

In some examples, the temperature of electroplating may be greater than or equal to 30°C and less than or equal to 70°C.

It should be noted that when the electroplating temperature is higher than 70°C, the higher temperature may result in decomposition of the electroplating gold plating solution, or the rapid volatilization of the electroplating gold plating solution, which is not favorable for the storage management of the electroplating gold plating solution. When the plating temperature is lower than 30°C, the lower temperature may cause the precipitation efficiency at the cathode to be reduced, and the plating layer may be uneven.

Exemplarily, the temperature of the plating may be 30°C, 35°C, 40°C, 45°C, 50°C, 55°C, 60°C or 70°C, and a random value within the range consisting of any two of the numerical values. Further preferably, the temperature of the plating may be within the range of 40°C-60°C.

It should be understood that the above-mentioned range values and specific values of said plating temperature are only illustrative and can be adjusted according to actual production and design requirements, and the present invention is not limited thereto.

In some examples, the current density of said electroplating may be greater than or equal to 0.1A/dm³ and less than or equal to 1.0A/dm³. It shall be comprehended that, when the current density of said electroplating goes beyond the aforementioned range, the electroplating gold plating solution may be decomposed or the plating layer may be uneven.

Exemplarily, the current density of said electroplating may be 0.1A/dm³, 0.2A/dm³, 0.3A/dm³, 0.4A/dm³, 0.5A/dm³, 0.7A/dm³, 0.9A/dm³, or 1.0A/dm³, and a random value within the range consisting of any two of the numerical values. More preferably, the current density of said electroplating may be within the range of 0.151A/dm³-0.951A/dm³. Further preferably, the current density of said electroplating may be within the range of 0.31A/dm³ to 0.851A/dm³.

It should be understood that the above-mentioned range values and specific values of the electroplating current density are only illustrative and can be adjusted according to actual production and design requirements, and the present invention is not limited thereto.

Exemplarily, the heat treatment may be an annealing treatment. For example, the electroplated semiconductor can be annealed under the temperature condition of 280°C for 60 min.

It should be understood that the specific conditions for the heat treatment are merely illustrative and can be adjusted according to actual production and design requirements, for example, the present invention is not limited thereto, and the specific condition does not impose limitations on the present invention.

In some examples, the hardness of the gold bump produced with the preparation method after heat treatment may be greater than or equal to 90HV and less than or equal to 120 HV

It shall be noted that the bonding performance of the electroplated gold is related to the hardness thereof, and the higher the hardness, the higher the pressure and temperature required during the bonding process, and the hardness of the electroplated gold after the heat treatment is generally controlled to be less than 120HV in view of the possible influence on the device performance. That is to say, the gold bumps manufactured based on the preparation method may have a high hardness after heat treatment under the premise of avoiding an influence on device performance, it is conducive to avoiding the short circuit and failure caused by deformation of the gold bumps during the thermocompression bonding process while reducing the size of gold bumps and the spacing between neighboring gold bumps.

The examples of the present invention also provide a gold bump produced based on the preparation method thereof. The gold bump after heat treatment may have a high hardness (for example, the hardness may be within the range of 90-120HV) and a regular shape.

The examples of the present invention further provide an electronic part comprising the aforementioned gold bump. The electronic part may be, for example, a radio frequency identification chip, an optical radar sensing chip, a liquid crystal driver chip, and other semiconductor components.

Further, the examples of the present invention provide an electronic device comprising the aforementioned electronic part. Examples of said electronic device may be a mobile phone, a tablet computer, an electronic reader, a personal computer (PC), a notebook computer, a personal digital assistant (PDA), an On-Board Equipment, a television (or a smart screen), a wearable device, a smartwatch, a smart wristband.

Embodiments of the present invention are further illustrated in the following examples. It shall be understood that a portion of the advantages of examples in the present invention will be formulated in the description below, and a portion of advantages is obvious based on the description or may be known by those skilled in the art through the implementation of examples of the present invention.

### [Example 1]

600mL of deionized water was added into a 2L beaker, 65g of hydroxyl ethylidene diphosphonic acid (HEDP) was then added, and after the HEDP was completely dissolved by stirring, the pH of a solution in the beaker was adjusted to 6.0 by using a potassium hydroxide solution, 30g of potassium oxalate and potassium aurous cyanide containing 8g of the gold element were subsequently added. After the solution was fully dissolved, the lead acetate with a lead element content of 6mg and 0.1g of catechol were further added, after the materials were completely dissolved, the liquid level of the plating solution was adjusted to 1L, and the pH of said plating solution was modified to 6.0 to obtain the electroplating gold plating solution.

During an electroplating process, platinum titanium was used as an anode, and a fresh nickel plating sheet was used as a cathode. The electroplating solution was heated to 40°C (i.e., electroplating temperature), the current density was adjusted to 0.5A/dm³, and the electroplating was performed for 110min to obtain a gold-plated part.

After the electroplating was finished, the gold-plated part was observed, wherein the surface of the plating layer was matt.

### [Example 2]

Example 2 differed from the method shown in Example 1 in that "30g of potassium oxalate" added in Example 1 was replaced with "15g of potassium oxalate", and "0.1g of catechol" added in Example 1 was replaced with "0.2g of catechol".

After the electroplating was finished, the gold-plated part was observed, wherein the surface of the plating layer was matt.

### [Example 3]

Example 3 differed from the method shown in Example 1 in that "0.1g of catechol" added in Example 1 was replaced with "0.5g of catechol".

After the electroplating was finished, the gold-plated part was observed, wherein the surface of the plating layer was matt.

### [Example 4]

Example 4 differed from the method shown in Example 1 in that "0.1g of catechol" added in Example 1 was replaced with "1.0g of sodium 4-hydroxybenzenesulfonate ".

After the electroplating was finished, the gold-plated part was observed, wherein the surface of the plating layer was matt.

### [Example 5]

Example 5 differed from the method shown in Example 1 in that "0.1g of catechol" added in Example 1 was replaced with "1.0g of 4-hydroxy benzaldehyde ".

After the electroplating was finished, the gold-plated part was observed, wherein the surface of the plating layer was matt.

### [Example 6]

Example 6 differed from the method shown in Example 1 in that "0.1g of catechol" added in Example 1 was replaced with "0.2g of 4-hydroxybenzoic acid ".

After the electroplating was finished, the gold-plated part was observed, wherein the surface of the plating layer was matt.

### [Example 7]

Example 7 differed from the method shown in Example 1 in that "0.1g of catechol" added in Example 1 was replaced with "0.5g of 3,4-dihydroxyl benzoic acid ".

After the electroplating was finished, the gold-plated part was observed, wherein the surface of the plating layer was matt.

### [Example 8]

Example 8 differed from the method shown in Example 1 in that "65g of HEDP" added in Example 1 was replaced with "65g of monopotassium phosphate", and "0.1g of catechol" added in Example 1 was replaced with "0.5g of 3,5-dihydroxyl benzoic acid".

After the electroplating was finished, the gold-plated part was observed, wherein the surface of the plating layer was matt.

### [Example 9]

Example 9 differed from the method shown in Example 1 in that "65g of HEDP" added in Example 1 was replaced with "65g of monopotassium phosphate", and "0.1g of catechol" added in Example 1 was replaced with "0.5 g of 3-hydroxypyridine ".

After the electroplating was finished, the gold-plated part was observed, wherein the surface of the plating layer was matt.

### [Example 10]

Example 10 differed from the method shown in Example 1 in that "65g of HEDP" added in Example 1 was replaced with "65g of monopotassium phosphate", "0.1g of catechol" added in Example 1 was replaced with "0.1g of gelatin", and the condition "the electroplating solution was heated to a temperature of 40°C" in Example 1 was replaced with "the electroplating solution was heated to a temperature of 60°C".

After the electroplating was finished, the gold-plated part was observed, wherein the surface of the plating layer was matt.

### [Example 11]

Example 11 differed from the method shown in Example 1 in that "65g of HEDP" added in Example 1 was replaced with "65g of monopotassium phosphate", "0.1g of catechol" added in Example 1 was replaced with "0.5g of gelatin", and the condition "the electroplating solution was heated to a temperature of 40°C" in Example 1 was replaced with "the electroplating solution was heated to a temperature of 60°C".

After the electroplating was finished, the gold-plated part was observed, wherein the surface of the plating layer was matt.

### [Example 12]

Example 12 differed from the method shown in Example 1 in that "0.1g of catechol" added in Example 1 was replaced with "0.1g of gelatin", and the condition "the electroplating solution was heated to a temperature of 40°C" in Example 1 was replaced with "the electroplating solution was heated to a temperature of 60°C".

After the electroplating was finished, the gold-plated part was observed, wherein the surface of the plating layer was matt.

### [Example 13]

Example 13 differed from the method shown in Example 1 in that "0.1g of catechol" added in Example 1 was replaced with "0.5g of gelatin", and the condition "the electroplating solution was heated to a temperature of 40°C" in Example 1 was replaced with "the electroplating solution was heated to a temperature of 60°C".

After the electroplating was finished, the gold-plated part was observed, wherein the surface of the plating layer was matt.

### [Example 14]

Example 14 differed from the method shown in Example 1 in that "0.1g of catechol" added in Example 1 was replaced with "0.05g of catechol".

After the electroplating was finished, the gold-plated part was observed, wherein the surface of the plating layer was matt.

### [Example 15]

Example 15 differed from the method shown in Example 11 in that "6 mg of lead acetate" added in Example 11 was replaced with "1 mg of lead acetate".

After the electroplating was finished, the gold-plated part was observed, wherein the surface of the plating layer was matt.

### [Example 16]

Example 16 differed from the method shown in Example 12 in that "0.1 g of gelatin" added in Example 12 was replaced with "0.01 g of gelatin".

After the electroplating was finished, the gold-plated part was observed, wherein the surface of the plating layer was matt.

### [Example 17]

Example 17 differed from the method shown in Example 10 in that "0.1 g of gelatin" added in Example 10 was replaced with "1 g of gelatin", and "6 mg of lead acetate" added in Example 10 was replaced with "8 mg of lead acetate".

After the electroplating was finished, the gold-plated part was observed, wherein the surface of the plating layer was matt.

### [Comparative Example 1]

Comparative Example 1 differs from the method shown in Example 1 in that "0.1g of catechol" added in Example 1 was replaced with "0 g of catechol".

After the electroplating was finished, the gold-plated part was observed, wherein the surface of the plating layer was matt.

### [Comparative Example 2]

Comparative Example 2 differs from the method shown in Example 12 in that "0.1g of gelatin" added in Example 12 was replaced with "0 g of gelatin".

After the electroplating was finished, the gold-plated part was observed, wherein the surface of the plating layer was matt.

The precipitation efficiencies of the electroplated parts of Examples 1-17 and Comparative Examples 1-2 were calculated, and the hardness of the plating layers before and after the heat treatment were measured, the results were as shown in Table 1. Wherein

Precipitation efficiency: after the electroplating process was finished, the precipitation efficiency of the electroplating gold plating solution was calculated by using a weighing method, wherein the precipitation efficiency denoted a ratio obtained by dividing the weight of the electroplated gold by the theoretical weight of the monovalent gold of the electric quantity passing through the electroplating process.

Coating hardness: the hardness of the plating layer was measured using a Vickers Hardness Tester. Specifically, a measuring indenter was held on the surface of the plating layer for 10 seconds using a load of 10 gf to test the hardness of the plating layer before and after the heat treatment, wherein the heat treatment conditions were as follows: the annealing treatment was performed at 280°C for 60 min.

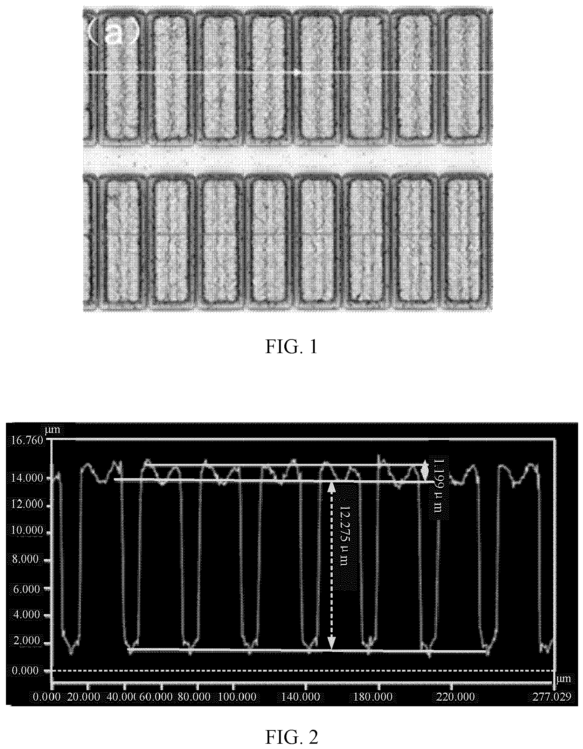

Electroplating was carried out in a Yamamoto plating vertical electroplating tank using the electroplating gold plating solution of Example 1 at a current density of 0.5A/dm³, an electroplating temperature of 40°C and an electroplating time of 40 min. The photograph of the obtained gold bump under a contour microscope at a magnification of 100 was shown in FIG. 1, and the corresponding height measurement result was shown in FIG. 2.

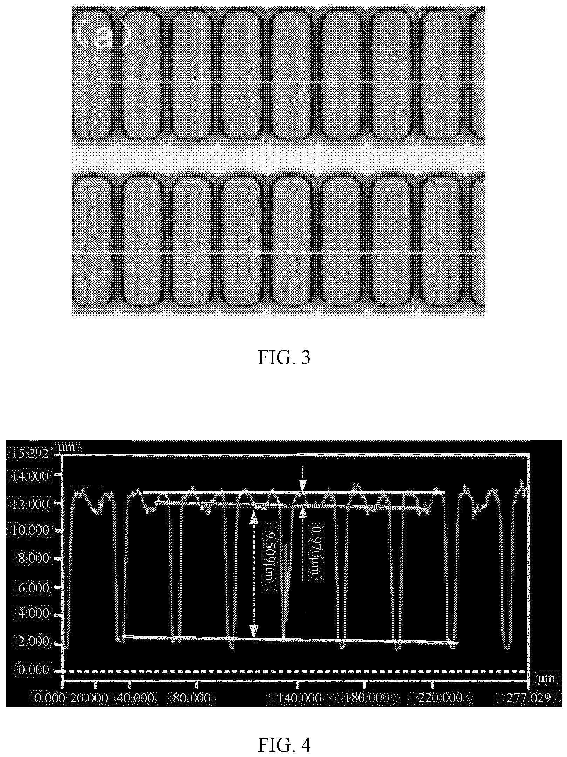

Electroplating was carried out in a Yamamoto plating vertical electroplating tank using the electroplating gold plating solution of Example 10 at a current density of 0.5A/dm³, an electroplating temperature of 65°C and an electroplating time of 30 min. The photograph of the obtained gold bump under a contour microscope at a magnification of 100 was shown in FIG. 3, and the corresponding height measurement result was shown in FIG. 4.

**Table 1**

| Examples | Example 1 | Example 2 | Example 3 | Example 4 | Example 5 | Example 6 | Example 7 | Example 8 | Example 9 | |
|---|---|---|---|---|---|---|---|---|---|---|
| Gold (potassium aurous cyanide), g/L | 8 | 8 | 8 | 8 | 8 | 8 | 8 | 8 | 8 | |
| Monopotassium phosphate, g/L | 0 | 0 | 0 | 0 | 0 | 0 | 0 | 65 | 65 | |
| HEDP, g/L | 65 | 65 | 65 | 65 | 65 | 65 | 65 | 0 | 0 | |
| Potassium oxalate, g/L | 30 | 15 | 30 | 30 | 30 | 30 | 30 | 30 | 30 | |
| Lead acetate, mg/L | 6 | 6 | 6 | 6 | 6 | 6 | 6 | 6 | 6 | |
| Catechol, g/L | 0.1 | 0.2 | 0.5 | 0 | 0 | 0 | 0 | 0 | 0 | |
| Sodium 4-hydroxy benzene sulfonate, g/L | 0 | 0 | 0 | 1.0 | 0 | 0 | 0 | 0 | 0 | |
| 4-hydroxy benzaldehyde, g/L | 0 | 0 | 0 | 0 | 1.0 | 0 | 0 | 0 | 0 | |
| 4-hydroxy benzoic acid, g/L | 0 | 0 | 0 | 0 | 0 | 0.2 | 0 | 0 | 0 | |
| 3,4-dihydroxy benzoic acid, g/L | 0 | 0 | 0 | 0 | 0 | 0 | 0.5 | 0 | 0 | |
| 3,5-dihydroxy benzoic acid, g/L | 0 | 0 | 0 | 0 | 0 | 0 | 0 | 0.5 | 0 | |
| 2,4,6-tri | 0 | 0 | 0 | 0 | 0 | 0 | 0 | 0 | 0.5 | |
| hydroxyl benzoic acid, g/L | | | | | | | | | | |
| Gelatin, g/L | 0 | 0 | 0 | 0 | 0 | 0 | 0 | 0 | 0 | |
| pH | 6 | 6 | 6 | 6 | 6 | 6 | 6 | 6 | 6 | |
| Temperature, °C | 40 | 40 | 40 | 40 | 40 | 40 | 40 | 40 | 40 | |
| Current density, A/dm³ | 0.5 | 0.5 | 0.5 | 0.5 | 0.5 | 0.5 | 0.5 | 0.5 | 0.5 | |
| Electroplating time, min | 110 | 110 | 110 | 110 | 110 | 110 | 110 | 110 | 110 | |
| Precipitation efficiency, % | 92 | 95 | 92 | 95 | 95 | 92 | 92 | 94 | 95 | |
| Hardness before annealing, HV | 94 | 110 | 109 | 101 | 98 | 98 | 103 | 97 | 95 | |
| Hardness after annealing, HV | 90 | 101 | 106 | 95 | 95 | 93 | 91 | 95 | 91 | |

| Examples | Examp le 10 | Examp le 11 | Examp le 12 | Examp le 13 | Examp le 14 | Examp le 15 | Examp le 16 | Examp le 17 | Comparati ve Example 1 | Comparati ve Example 2 |
|---|---|---|---|---|---|---|---|---|---|---|
| Gold (potassium aurous cyanide), g/L | 8 | 8 | 8 | 8 | 8 | 8 | 8 | 8 | 8 | 8 |
| Monopotassi um phosphate, g/L | 65 | 65 | 0 | 0 | 0 | 65 | 0 | 65 | 0 | 0 |
| HEDP, g/L | 0 | 0 | 65 | 65 | 65 | 0 | 65 | 0 | 65 | 65 |
| Potassium oxalate, g/L | 30 | 30 | 30 | 30 | 30 | 30 | 30 | 30 | 30 | 30 |
| Lead acetate, mg/L | 6 | 6 | 6 | 6 | 6 | 1 | 6 | 8 | 6 | 6 |
| Catechol, g/L | 0 | 0 | 0 | 0 | 0.05 | 0 | 0 | 0 | 0 | 0 |
| Sodium 4-hydroxy benzene sulfonate, g/L | 0 | 0 | 0 | 0 | 0 | 0 | 0 | 0 | 0 | 0 |
| 4-hydroxy benzaldehyd | 0 | 0 | 0 | 0 | 0 | 0 | 0 | 0 | 0 | 0 |
| e, g/L | | | | | | | | | | |
| 4-hydroxy benzoic acid, g/L | 0 | 0 | 0 | 0 | 0 | 0 | 0 | 0 | 0 | 0 |
| 3,4-dihydrox y benzoic acid, g/L | 0 | 0 | 0 | 0 | 0 | 0 | 0 | 0 | 0 | 0 |
| 3,5-dihydrox y benzoic acid, g/L | 0 | 0 | 0 | 0 | 0 | 0 | 0 | 0 | 0 | 0 |
| 2,4,6-tri hydroxyl benzoic acid, g/L | 0 | 0 | 0 | 0 | 0 | 0 | 0 | 0 | 0 | 0 |
| Gelatin, g/L | 0.1 | 0.5 | 0.1 | 0.5 | 0 | 0.5 | 0.01 | 1 | 0 | 0 |
| pH | 6 | 6 | 6 | 6 | 6 | 6 | 6 | 6 | 6 | 6 |
| Temperature, °C | 60 | 60 | 60 | 60 | 40 | 60 | 60 | 60 | 40 | 60 |
| Current density, A/dm³ | 0.5 | 0.5 | 0.5 | 0.5 | 0.5 | 0.5 | 0.5 | 0.5 | 0.5 | 0.5 |
| Electroplatin g time, min | 110 | 110 | 110 | 110 | 110 | 110 | 110 | 110 | 110 | 110 |
| Precipitation efficiency, % | 96 | 97 | 98 | 98 | 93 | 97 | 98 | 99 | 96 | 98 |
| Hardness before annealing, HV | 136 | 131 | 103 | 103 | 81 | 146 | 93 | 137 | 104 | 76 |
| Hardness after annealing, HV | 118 | 114 | 92 | 98 | 78 | 83 | 84 | 120 | 70 | 50 |

With reference to the data in Table 1, gold bumps obtained by electroplating with the electroplating gold plating solution in Examples 1-17 of the present invention not only have a high hardness before the heat treatment but also maintains a high hardness after the heat treatment, both are higher than the hardness of gold bumps in Comparative Examples 1 and 2 after the heat treatment.

Specifically, as can be seen by combining the Comparative Example 1 and Example 1, the hardness of the gold bump after heat treatment can be significantly improved by adding catechol into the electroplating gold plating solution, so that the gold bump still has a high hardness of 90 HV As can be seen with reference to Comparative Example 2 and Example 12, the addition of gelatin into the electroplating gold plating solution can not only significantly increase the hardness of gold bump before the heat treatment, such that the gold bump before the heat treatment has a high hardness of 103HV, but also significantly improve the hardness of gold bump after the heat treatment, so that the gold bump after the heat treatment still has a high hardness of 92HV.

In addition, as can be seen with reference to Example 14 and Example 1, when the amount of catechol added into the electroplating gold plating solution is only 0.05g because the content is too low, it is not beneficial to that catechol performs an effect of increasing the hardness of gold bump after the heat treatment. As can be seen from the combination of Example 15 and Example 11, the lead acetate is added into the electroplating solution, the hardness of said gold bump after heat treatment is 83 HV when the content of lead element is 1mg, and the hardness of said gold bump after heat treatment is 114 HV when the content of lead element is 6mg, which indicates that lead acetate and gelatin can produce the synergistic effect to increase the hardness of gold bump after the heat treatment. As can be seen with reference to Example 16 and Example 12, when the amount of gelatin added into the electroplating gold plating solution is only 0.01g because the content is too low, it is not beneficial to that gelatin performs an effect of increasing the hardness of gold bump before and after the heat treatment. The gold bumps produced by electroplating with the electroplating gold plating solution in Examples 1-13 of the present invention not only have a high hardness before the heat treatment but also can maintain a high hardness greater than or equal to 90HV after the heat treatment.

With reference to FIG. 1 and FIG. 3, the gold bumps produced with the electroplating gold plating solution in Example 1 and Example 10 do not have the defects of leakage plating and infiltration plating on the photograph, and there is no dissolution or rupture of the photoresist. The gold bumps have a relatively regular shape, the golden tumor or pinhole is not observed on the surface of the gold bumps. The surface of said gold bump is relatively flat and has no inclination. As shown in FIG. 2, the gold bump produced with the electroplating gold plating solution of Example 1 has a height of 12.275µm and a front-end height difference of 1.199µm. As shown in FIG. 4, the gold bump produced with the electroplating gold plating solution of Example 10 has a height of 9.509µm and a front-end height difference of 0.970µm. Compared with the front-end height difference of 1.199µm shown in FIG. 2, the front-end height difference illustrated in FIG. 4 is decreased, demonstrating that the electroplating gold plating solution provided by the examples of the present invention also has a certain leveling ability.

To sum up, as can be seen from Table 1 and FIGS. 1-4, in the examples of the present invention, a phenolic compound and/or gelatin is added into the electroplating gold plating solution, and the phenolic compound and/or gelatin produce synergy with other components, so that the gold bump prepared with the electroplating gold plating solution can still have a high hardness (e.g., 90-120 HV) after the heat treatment, and the produced gold bump has a regular shape, which conforms to the specification required for production. Therefore, the present invention is beneficial to reducing the size of the gold bump and the spacing between adjacent gold bumps and avoiding the short circuit and failure of the gold bump caused by the deformation during the thermocompression bonding process.

The above content is only the specific embodiments of the present invention, but the protection scope of the present invention is not limited thereto, any change or substitution within the technical scope disclosed in the present invention can be easily envisaged by those skilled in the art who are familiar with common knowledge in the technical field shall fall into the technical scope of the present invention. As a result, the protection scope of the present invention shall be subject to the protection scope of the claims.

## Claims

1. An electroplating gold plating solution, is **characterized in that** it comprises a gold cyanide salt, a conductive salt, an oxalate, a lead-containing compound, and an additive,
the additive comprises a phenolic compound and/or a gelatin, and the general formula of the phenolic compound is as shown in the following formula (1): 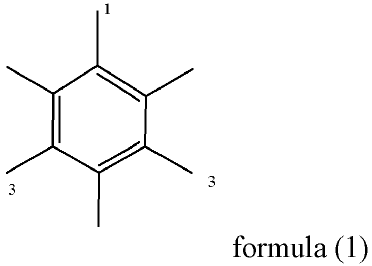 in the formula (1), at least one of R₁, R₂, R₃, R₄, R₅, and R₆ is selected from hydroxyl, and the rest are each independently any one selected from the group consisting of hydrogen atom, halogen group, carboxyl, aldehyde group, sulfonic acid group, amino, and alkyl.

2. The electroplating gold plating solution according to claim 1, wherein the phenolic compound comprises at least one selected from the group consisting of phenol, catechol, resorcinol, hydroquinone, 2-hydroxybenzoic acid, 3-hydroxybenzoic acid, 4-hydroxybenzoic acid, 4-hydroxybenzaldehyde, 4-hydroxybenzenesulfonic acid, 2,3-dihydroxybenzoic acid, 2,4-dihydroxybenzoic acid, 2,5-dihydroxybenzoic acid, 2,6-dihydroxybenzoic acid, 3,4-dihydroxybenzoic acid, 3,5-dihydroxybenzoic acid, 3,4,5-trihydroxybenzoic acid, and 2,4, 6-trihydroxybenzoic acid.

3. The electroplating gold plating solution according to claim 1 or 2, wherein the electroplating gold plating solution satisfies at least one of the following conditions:
the concentration of said phenolic compound in the electroplating gold plating solution is greater than or equal to 0.05g/L and less than or equal to 2.0g/L;
the concentration of gelatin in the electroplating gold plating solution is greater than or equal to 0.05g/L and less than or equal to 2.0 g/L.

4. The electroplating gold plating solution according to any one of claims 1-3, wherein the gold cyanide salt comprises at least one of potassium aurous cyanide, sodium aurous cyanide, and ammonium aurous cyanide;
preferred, the gold cyanide salt is used in an amount such that the concentration of gold ions in the electroplating gold plating solution is greater than or equal to 1g/L and less than or equal to 20 g/L.

5. The electroplating gold plating solution according to any one of claims 1-4, wherein the conductive salt comprises a phosphate and/or an organophosphonic acid;
wherein the phosphate comprises at least one of potassium phosphate, sodium phosphate, and ammonium phosphate,
the organic phosphonic acid comprises at least one of hydroxyl ethylidene diphosphonic acid, aminotrimethylene phosphonic acid, and ethylenediamine tetramethylene phosphonic acid;
preferred, the concentration of said conductive salt in the electroplating gold plating solution is greater than or equal to 10 g/L and less than or equal to 100 g/L.

6. The electroplating gold plating solution according to any one of claims 1-5, wherein the oxalate comprises at least one of potassium oxalate, sodium oxalate, and ammonium oxalate;
preferred, the concentration of said oxalate in the electroplating gold plating solution is greater than or equal to 5g/L and less than or equal to 80g/L.

7. The electroplating gold plating solution according to any one of claims 1-6, wherein the lead-containing compound comprises at least one of lead acetate, lead nitrate, lead citrate, and lead sulfate;
preferred, the concentration of said lead-containing compound is calculated in terms of lead, the concentration of said lead-containing compound in the electroplating gold plating solution is greater than or equal to 2ppm and less than or equal to 15ppm.

8. The electroplating gold plating solution according to any one of claims 1-7, further comprises a hydrogen ion concentration index pH additive, which is used for adjusting the pH of the electroplating gold plating solution;
preferred, the pH of said electroplating gold plating solution is greater than or equal to 5 and less than or equal to 7.

9. An use of the electroplating gold plating solution according to any one of claims 1-8, wherein the electroplating gold plating solution is applied in the gold plating of the semiconductor.

10. A preparation method for gold bump, is **characterized in that** the preparation method comprises the following steps:
placing a semiconductor in the electroplating gold plating solution according to any one of claims 1-8 for electroplating;
carrying out heat treatment on the electroplated semiconductor to form a gold bump on the surface of said semiconductor.

11. The preparation method according to claim 10, wherein the method satisfies at least one of the following conditions:
the electroplating temperature is greater than or equal to 30°C and less than or equal to 70°C;
the current density of said electroplating is greater than or equal to 0.1A/dm³ and less than or equal to 1.0A/dm³.

12. The preparation method according to claims 10 or 11, wherein the hardness of said gold bump after heat treatment is greater than or equal to 90HV and less than or equal to 120 HV.

13. A gold bump, is **characterized in that** the gold bump is produced with the preparation method according to any one of claims 10-12.

14. An electronic part, is **characterized in that** the electronic part comprises a gold bump according to claim 13.

15. An electronic device, is **characterized in that** the electronic device comprises the electronic part according to claim 14.
